# EUROPÄISCHE PATENTSCHRIFT

(11) **EP 0 629 207 B1**
(45) Veröffentlichungstag und Bekanntmachung des Hinweises auf die Patenterteilung: **04.06.1997**
(21) Anmeldenummer: 93904022.6
(22) Anmeldetag: 26.02.1993
(51) Int. Cl.: C07H 15/04

(54) **VERFAHREN ZUR HERSTELLUNG VON ALKYL- UND/ODER ALKENYLOLIGOGLUCOSIDEN**
PROCESS FOR PREPARING ALKYL AND/OR ALKENYL OLIGOGLUCOSIDES
PROCEDE DE FABRICATION D'ALKYLOLIGOGLUCOSIDES ET/OU D'ALCENYLOLIGOGLUCOSIDES

(30) Priorität: 06.03.1992 DE 4207101
(43) Veröffentlichungstag der Anmeldung: 21.12.1994
(73) Patentinhaber: Henkel Kommanditgesellschaft auf Aktien, 40191 Düsseldorf (DE)
(72) Erfinder: CARDUCK, Franz-Josef, D-5657 Haan (DE); SCHULZ, Paul, D-5600 Wuppertal 1 (DE); ESKUCHEN, Rainer, D-4000 Düsseldorf 13 (DE)
(86) Internationale Anmeldenummer: EP9300444
(87) Internationale Veröffentlichungsnummer: WO9318046

(56) Entgegenhaltungen:
- EP-A- 0 319 616
- EP-A- 0 362 671
- US-A- 2 390 507

## Beschreibung

### Gebiet der Erfindung

Die Erfindung betrifft ein Verfahren zur Herstellung von Alkyl- und/oder Alkenyloligoglucosiden durch Trocknung einer wäßrigen Glucose/Fettalkohol-Suspension und anschließende Acetalisierung der entwässerten Mischung in Gegenwart saurer Katalysatoren.

### Stand der Technik

Alkyloligoglykoside stellen wichtige nichtionische Tenside für eine Vielzahl von Anwendungsgebieten dar. Zu ihrer Herstellung geht man in der Regel von Aldosen - in der Regel Glucose - aus, die in Gegenwart saurer Katalysatoren mit Fettalkoholen acetalisiert werden (Direktverfahren). Zur Erzielung hoher Ausbeuten innerhalb hinreichend kurzer Reaktionszeiten ist es jedoch vorteilhaft, daß die Umsetzung in Abwesenheit von Wasser durchgeführt wird. Im einzelnen bedeutet dies, daß für die Acetalisierung nur solche Einsatzstoffe in Betracht kommen, die wasserfrei sind, d. h. deren Restwassergehalt 2 Gew.-% nicht übersteigt. Stellvertretend für das umfangreiche Schrifttum sei hier auf die Internationale Patentanmeldung **WO 90/3977** verwiesen.

Die Verwendung von Reinstoffen, wie beispielsweise reiner wasserfreier Glucose, belastet die Kosten der Herstellung von Alkyloligoglucosiden jedoch in einem solchen Maße, daß eine Wirtschaftlichkeit in vielen Fällen nicht mehr gegeben ist. Es hat in der Vergangenheit daher nicht an Versuchen gemangelt, technische Glucose auf der Basis preiswerter wäßriger Glucosesirupe einzusetzen, die zuvor bis auf das erforderliche Maß getrocknet worden waren [**EP-A1 0 319 616**]. Als entscheidender Nachteil wurde dabei jedoch erkannt, daß übliche Trocknungsverfahren stets Einfluß auf Qualität und Zusammensetzung der wasserfreien Produkte nehmen und insbesondere zu einem unerwünschten Anstieg des Gehaltes an Oligo- und Polyzuckern beitragen können.

Die Aufgabe der Erfindung bestand somit darin, ein neues Verfahren zur Herstellung von Alkyl- und/oder Alkenyloligoglucosiden zur Verfügung zu stellen, das frei von den geschilderten Nachteilen ist.

### Beschreibung der Erfindung

Gegenstand der Erfindung ist ein Verfahren zur Herstellung von Alkyl- und/oder Alkenyloligoglucosiden, das sich dadurch auszeichnet, daß man
a) wäßrige Glucosesirupe und Fettalkohole in einem Turbinentrockner mit rotierenden Einbauten bis auf einen Restwassergehalt von 0,05 bis 0,3 Gew.-% entwässert und
b) die resultierenden Glucose/Fettalkohol-Suspensionen in Gegenwart saurer Katalysatoren in an sich bekannter Weise acetalisiert.

Überraschenderweise wurde gefunden, daß bei der Entwässerung von Mischungen wäßriger Glucosesirupe mit Fettalkoholen in einem Turbinentrockner wasserfreie Suspensionen erhalten werden, deren Gehalt an Oligo- bzw. Polyzuckern gegenüber dem Ausgangsmaterial nicht nachteilig erhöht ist.

Unter **Glucosesirupe** sind raffinierte wäßrige Lösungen von D-Glucose, Maltose und höheren Polymeren der Glucose (Oligosaccharide, Dextrine) zu verstehen, die durch saure Hydrolyse oder enzymatischen Abbau von Stärke hergestellt werden. Vorzugsweise finden Glucosesirupe Verwendung, die einen Feststoffanteil von 50 bis 85, vorzugsweise 75 bis 80 Gew.-% und einen DP1 Grad (monomerer Glucosegehalt) von 80 bis 99, vorzugseise 92 bis 97 Gew.-% - bezogen auf den Feststoff - aufweisen.

Als **Fettalkohole** kommen primäre Alkohole der Formel (I) in Betracht,

R¹OH (I)

in der R¹ für lineare oder verzweigte Alkyl- und/oder Alkenylreste mit 6 bis 22 Kohlenstoffatomen steht. Typische Beispiele sind Capronalkohol, Caprylalkohol, Caprinalkohol, Laurylalkohol, Myristylalkohol, Cetylalkohol, Palmoleylalkohol, Stearylalkohol, Oleylalkohol, Elaidylalkohol, Petroselinylalkohol, Arachylalkohol, Gadoleylalkohol, Behenylalkohol und/oder Erucylalkohol sowie technische Schnitte, die diese Alkohole in unterschiedlichen Mischungsverhältnissen enthalten können. Bevorzugt ist ein technischer Fettalkoholschnitt auf Basis von gehärtetem Kokosöl mit 12 bis 18, insbesondere 12 bis 14 Kohlenstoffatomen.

Das molare Einsatzverhältnis von Glucosesirup zu Fettalkohol kann 1 : 2 bis 1 : 10, vorzugsweise 1 : 3 bis 1 : 6 betragen, wobei das Verhältnis auf den Glucoseanteil im Sirup zu beziehen ist.

Unter **Turbinentrocknern** sind zylindrische Trockenapparaturen, vorzugsweise in horizontaler Bauweise zu verstehen, in denen rotierende Einbauten mit hoher Drehzahl für eine feine Verteilung des Trockengutes sorgen. In einer bevorzugten Ausführungsform handelt es sich bei diesen Einbauten beispielsweise um Flügel, Schaufeln oder Paddeln, die auf einer rotierenden Welle (Umfangsgeschwindigkeit 5 bis 25, vorzugsweise 10 bis 20 m/s) angebracht sind. Die eigentliche Trocknung findet bei Wandtemperaturen von 100 bis 180°C und Gasphasentemperaturen von 150 bis 220°C vorzugsweise in Gegenwart von Luft, Inertgasen wie beispielsweise Stickstoff oder überhitztem Wasserdampf statt, wobei der Wärmeübergang durch Konvektion sowie die beheizte Wandung des Trockners erfolgt. Im Hinblick auf die Herstellung wasserfreier Glucose/Fettal- kohol-Suspensionen hat sich eine Temperatur von 120 bis 180°C als optimal und ein verminderter Druck von 20 bis 300, vorzugsweise 50 bis 100 mbar erwiesen.

Da die aufgeheizte Luft bzw. das aufgeheizte Inertgas gleichzeitig mit dem zu trocknenden feuchten Produkt in den Trockner eingebracht wird, findet eine augenblickliche Verdampfung des Wassers statt. Aufgrund der hohen Verdampfungswärme von Wasser führt dies zu einem temperaturstabilisierenden Effekt, so daß die Trocknung auch bei hohen Temperaturen erfolgen kann, ohne daß es zu einer Zersetzung temperaturlabiler Produkte kommt.

Besondere Merkmale der erfindungsgemäß einzusetzenden Turbinentrockner sind somit die kurze Verweilzeit, das enge Verweilzeitspektrum und die hohe Temperaturstabilisierung, die eine möglichst schonende Behandlung des Trockengutes, gerade im Hinblick auf Zusammensetzung und Farbe, sicherstellen.

Die Abtrennung des Trockengutes von der Gasphase kann beispielsweise in einem Vakuum-Abtrennbehälter erfolgen. Um Produktverluste möglichst gering zu halten, empfiehlt es sich ferner, das Abgas beispielsweise über eine beheizten Kolonne zu leiten, Anteile an mitgerissenem Fettalkohol zu kondensieren und in die Suspension zurückzuführen.

Die nach dem erfindungsgemäßen Verfahren erhältlichen wasserfreien Glucose/Fettalkohol-Suspensionen weisen einen Rest-Wassergehalt von 0,1 bis 2 Gew.-% auf. Sie können in Gegenwart saurer Katalysatoren, beispielsweise p-Toluolsulfonsäure, in an sich bekannter Weise zu den entsprechenden Alkyl- und/oder Alkenyloligoglucosiden acetalisiert werden.

Die folgenden Beispiele sollen den Gegenstand der Erfindung näher erläutern, ohne ihn darauf einzuschränken.

### Beispiele

### A) Herstellung einer wasserfreien Glucose/Fettalkohol-Suspension

Die Herstellung der Suspension wurde in einem horizontal angeordneten Turbo-Trockner (Typ ES 2050, Fa.Vomm, Mailand, Italien, Turbinendurchmesser 340 mm, Turbinenlänge 2,4 m) durchgeführt, in dem eine mit Schaufeln bzw. Flügeln besetzte Welle mit hoher Drehzahl rotierte.

### Einsatzstoffe:

- a1): Glucosesirup
Feststoffanteil : 75 Gew.-%
DP1-Gehalt(*) : 95 Gew.-%
(*) Monomerer Glucosegehalt bezogen auf Feststoff
- a2): C_{12/14}-Kokosfettalkohol
(Lorol^{(R)} Spezial, Fa.Henkel KGaA, Düsseldorf).

Die Einsatzstoffe wurden getrennt auf eine Temperatur von 60°C vorgeheizt. Zunächst wurde der Fettalkohol über eine Kolbenpumpe in den "Kopf" des Turbinenmischers gefördert und anschließend - auf der Längsachse des Trockners betrachtet - in einigem Abstand der Glucosesirup über eine zweite Kolbenpumpe eindosiert; das molare Einsatzverhältnis Glucosesirup : Fettalkohol betrug dabei 1 : 4,5 - bezogen auf den Glucoseanteil im Sirup. Bei einer Umdrehungsgeschwindigkeit von 1000 Upm wurde die Mischung in einem heißen, turbulenten Luftstrom fein verteilt und gleichzeitig entwässert.

Die Trocknungstemperatur lag bei 160 bis 180°C und wurde zum einen durch Konvektion und zum anderen über den beheizten Mantel des Trockners übertragen. Am Mischeraustritt wurde ein Druck von 100 mbar eingestellt.

Am Ausgang des Mischers wurde der Glucose/Fettalkohol-Slurry in einen Vakuum-Abtrennbehälter ausgetragen und die Gasphase - enthaltend Wasserdampf und mitgerissenen Fettalkohol - über eine beheizte Kolonne und durch einen beheizten Wärmetauscher geführt. Der Fettalkohol wurde kondensiert und zurückgeführt, während der Wasserdampf in einem nachgeschalteten Kondensator niedergeschlagen wurde.

Die resultierende Glucose/Fettalkohol-Suspension wies einen Rest-Wassergehalt von 0,1 Gew.-% auf.

### B. Acetalisierung

In einem 2-1-Dreihalskolben mit Rührer, Destillationsaufsatz und Innenthermometer wurden 1050 g der entwässerten Glucose/ Fettalkohol-Suspension aus A) vorgelegt und unter einem verminderten Druck von ca. 20 mbar auf 110°C erhitzt.

Anschließend wurde die Reaktionsmischung mit 0,1 bis 0,5 Gew.-% - bezogen auf die Glucose - p-Toluolsulfonsäure in Form einer 5 gew.-%igen Lösung in Kokosfettalkohol versetzt. Zur Verlagerung des Gleichgewichtes wurde das Reaktionswasser kontinuierlich abdestilliert und die Reaktion abgebrochen, nachdem die Wasserabscheidung beendet war und der Restgehalt an nicht umgesetzter Glucose in der Mischung weniger als 0,1 Gew.-% - bezogen auf die Ausgangsmenge - betrug. Danach wurde die Reaktionsmischung mit Magnesiumoxid neutralisiert und der überschüssige Kokosfettalkohol unter vermindertem Druck (ca. 1 mbar) und einer Temperatur von 180°C mit Hilfe eines Dünnschichtverdampfers abgetrennt.

## Patentansprüche

1. Verfahren zur Herstellung von Alkyl- und/oder Alkenyloligoglucosiden, **dadurch gekennzeichnet**, daß man
a) wäßrige Glucosesirupe und Fettalkohole in einem Turbinentrockner mit rotierenden Einbauten bis auf einen Restwassergehalt von 0,05 bis 0,3 Gew.-% entwässert und
b) die resultierende Glucose/Fettalkohol-Suspensionen in Gegenwart saurer Katalysatoren in an sich bekannter Weise acetalisiert.

2. Verfahren nach Anspruch 1, **dadurch gekennzeichnet**, daß man Glucosesirup mit einem Feststoffanteil von 50 bis 85 Gew.-% und einem DP1-Grad (monomerer Glucosegehalt) von 80 bis 99 Gew.-% - bezogen auf den Feststoff - einsetzt.

3. Verfahren nach Anspruch 1, **dadurch gekennzeichnet**, daß man Fettalkohole der Formel (I) einsetzt,
R¹OH (I)
in der R¹ für einen linearen oder verzweigten Alkyl- und/oder Alkenylrest mit 6 bis 22 Kohlenstoffatomen steht.

4. Verfahren nach Anspruch 1, **dadurch gekennzeichnet**, daß man Glucosesirup und Fettalkohol im molaren Verhältnis 1 : 2 bis 1 : 10 - bezogen auf den Glucoseanteil im Sirup - einsetzt.

5. Verfahren nach Anspruch 1, **dadurch gekennzeichnet**, daß man die Entwässerung bei einer Temperatur von 120 bis 200°C durchführt.

6. Verfahren nach Anspruch 1, **dadurch gekennzeichnet**, daß man die Entwässerung in Gegenwart von Luft, Inertgas und/oder überhitztem Wasserdampf durchführt.

## Claims

1. A process for the production of alkyl and/or alkenyl oligoglucosides, **characterized in that**
a) aqueous glucose sirups and fatty alcohols are dried in a turbo dryer with rotating fittings to a residual water content of 0.05 to 0.3% by weight and
b) the resulting glucose/fatty alcohol suspensions are acetalized in known manner in the presence of acidic catalysts.

2. A process as claimed in claim 1, **characterized in that** glucose sirup with a solids content of 50 to 85% by weight and a DP1 degree (monomeric glucose content) of 80 to 99% by weight, based on the solids, is used.

3. A process as claimed in claim 1, **characterized in that** fatty alcohols corresponding to formula (I):
R¹OH (I)
in which R¹ is a linear or branched alkyl and/or alkenyl radical containing 6 to 22 carbon atoms,
are used.

4. A process as claimed in claim 1, **characterized in that** glucose sirup and fatty alcohol are used in a molar ratio of 1:2 to 1:10, based on the glucose in the sirup.

5. A process as claimed in claim 1, **characterized in that** drying is carried out at a temperature of 120 to 200°C.

6. A process as claimed in claim 1, **characterized in that** drying is carried out in the presence of air, inert gas and/or superheated steam.

## Revendications

1. Procédé de fabrication d'alkyloligoglucosides et/ou d'alcényloligoglucosides, **caractérisé en ce que** l'on
a) déshydrate des sirops de glucose aqueux et des alcools gras dans un turbosécheur comportant des déflecteurs en rotation jusqu'à une teneur résiduelle en eau de 0,05 à 0,3 % en poids et
b) acétalise les suspensions de glucose et d'alcool gras résultantes, en présence de catalyseurs acides, d'une manière connue en soi.

2. Procédé selon la revendication 1, **caractérisé en ce que** l'on utilise un sirop de glucose avec une proportion de matière solide de 50 à 85 % en poids et un degré DP1 (teneur en glucose monomère) de 80 à 99 % en poids par rapport à la matière solide.

3. Procédé selon la revendication 1, **caractérisé en ce que** l'on utilise des alcools gras de la formule (I)
R¹OH (I)
dans laquelle R¹ représente un radical alkyle ou alcényle linéaire ou ramifié comportant 6 à 22 atomes de carbone.

4. Procédé selon la revendication 1, **caractérisé en ce que** l'on utilise le sirop de glucose et l'alcool gras dans un rapport molaire de 1:2 à 1:10 (par rapport à la proportion de glucose dans le sirop).

5. Procédé selon la revendication 1, **caractérisé en ce que** l'on opère la déshydratation à une température de 120 à 200 °C.

6. Procédé selon la revendication 1, **caractérisé en ce que** l'on opère la déshydratation en présence d'air, de gaz inerte et/ou de vapeur d'eau surchauffée.
